# EUROPEAN PATENT APPLICATION

(11) **EP 4 803 001 A1**
(43) Date of publication of application: **09.09.2026**
(21) Application number: 26192508.5
(22) Date of filing: 05.01.2023
(51) Int. Cl.: A61B 5/1473

(54) **APPLICATOR FOR MEDICAL DEVICE**

(30) Priority: 12.01.2022 KR 20220004408
(62) Document number(s) of the earlier application(s) in accordance with Art. 76 EPC: 23740396.9 / 4 437 958
(71) Applicant: I-SENS, INC., Seoul 06646 (KR)
(72) Inventor: CHOI, Hyun Ho, 06646 Seoul (KR); RYU, Goang Yel, 06646 Seoul (KR); WANG, Ji Hoon, 06646 Seoul (KR)
(74) Representative: BCKIP Part mbB

(57) **Abstract**

An applicator according to the present invention is an applicator for a medical device, for attaching a medical device to a user's skin, and comprises: an applicator body; an insertion unit installed on the applicator body to insert into the user's skin an insertion part of the medical device including the insertion part of which at least a part is inserted into the user's skin; an actuating part installed on the applicator body to allow at least a part thereof to move from an inactive position to an active position at which the insertion unit can be actuated; and a backstop part which locks at least a part of the actuating part to the active position to prevent the at least a part of the actuating part from returning to the inactive position from the active position.

## Description

### TECHNICAL FIELD

The present disclosure relates to an applicator for a medical device, and more specifically, to an applicator and an applicator assembly for a medical device to attach a medical device, which is used by being attached to the skin of a user, to the skin of the user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed and used, and therefore easily perform the management of diabetics and responses to an emergency situation. In order to minimize the pain and resistance to blood collection, the continuous glucose monitoring systems can measure blood glucose continuously after inserting a needle-shaped transcutaneous sensor into areas where pain is relatively less, such as the stomach and arms.

The continuous blood glucose measurement system consists of a sensor unit including a transcutaneous sensor that is inserted into the skin of a user and measures blood glucose levels within the body, a transmitter for transmitting blood glucose levels measured by a transcutaneous sensor and a terminal for outputting the received blood glucose levels.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

### SUMMARY

### Technical Problem

The present disclosure is developed in consideration of the above-described points, and the purpose of the present disclosure is to minimize the user's additional work to attach the medical device to the skin by enabling the medical device to be manufactured in an assembled state, and provide an applicator and an applicator assembly for a medical device that can be conveniently used to attach a medical device to the skin.

Another purpose of the present disclosure is to provide an applicator and an applicator assembly for a medical device that prevent reuse of the applicator after attaching the medical device to the skin and enable safe disposal of the applicator.

### Solution to Problem

To accomplish the purpose of the present disclosure described above, an applicator for a medical device for attaching the medical device to skin of a user according to the present disclosure may comprise: an applicator body; an insertion unit installed at the applicator body to insert an insertion portion of the medical device, which includes an insertion portion configured to be at least partially insertable into the skin, into the skin of the user; an operation portion installed at the applicator body such that at least a part of the operation portion is movable from a deactivated position to an activated position operating the insertion unit; and a backstop portion configured to lock at least the part of the operation portion in the activated position so as to prevent at least the part of the operation portion from returning from the activated position to the deactivated position.

The backstop portion may comprise a backstop detent provided at one of the applicator body and the operation portion, and a backstop member provided at an other one of the applicator body and the operation portion so as to be engaged with the backstop detent after the operation portion moves to the activation position.

The backstop member may comprise a backstop member body provided to be elastically deformable at the other one of the applicator body and the operation portion, and a backstop member snap provided at one side of the backstop member body so as to be engaged with the backstop detent.

The one of the applicator body and the operation portion may be provided with an accommodation groove in which at least a portion of the backstop member snap is accommodated when the operation portion is located in the deactivated position.

The operation portion may be configured to be movable in a direction of intersecting a direction of movement of the insertion portion by the insertion unit.

The insertion unit may include a shuttle to which the insertion portion is detachably coupled, and movably installed at the applicator body to move the insertion portion from a first position where the insertion portion is away from the skin of the user to a second position where the insertion portion is inserted into the skin of the user, and a forward elastic member configured to provide a moving force to the shuttle to move the shuttle to the second position, and the operating portion may include a button portion exposed to an outside of the applicator body; and a stopper portion configured to be movable from the deactivated position to the activated position by the button portion, be engageable with the shuttle in the deactivated position to prevent the shuttle from moving from the first position, and be disengageable from the shuttle in the activated position to allow the shuttle to move to the second position, and the backstop portion may be configured to lock the stopper portion in the activated position.

The insertion unit may include a needle configured to be insertable into the skin of the user together with the insertion portion, a carrier coupled to the needle and movably installed to the shuttle, and a backward elastic member configured to apply an elastic force from the second position to the first position with respect to the carrier, and wherein the shuttle includes a fixing groove into which a portion of the backward elastic member is inserted, a contact surface where a portion of the backward elastic member, which is inserted into the fixing groove, contacts, and a barrier portion protruding from the contact surface to prevent the portion of the backward elastic member in contact with the contact surface from falling out from the fixing groove.

The applicator according to the present disclosure may comprise: a locking hook tiltably coupled to the applicator body to be engaged with the base unit to detachably couple the base unit, attachable to the skin of the user, to the applicator body for the insertion portion to be coupled, wherein the operation portion is configured to restrain movement of the locking hook so that the locking hook is not tilted when at least the part of the operation portion is located in the deactivated position, and release restraint from the locking hook when at least the part of the operation portion is located in the activated position.

The operation portion may include: a button portion exposed to an outside of the applicator body; a stopper portion configured to move in a direction of intersecting a moving direction of the insertion portion by the button portion; and a stopper protrusion extending from the stopper portion toward the locking hook to contact one side of the locking hook and restrain the movement of the locking hook.

The applicator according to the present disclosure may comprise: a locking body installed at the applicator body to be engaged with at least the part of the operation portion or disengaged from at least the part of the operation portion; and a moving tab linearly movably coupled to the applicator body to move from a locking position, where at least a portion of the moving tab is located inside the applicator body, to a removal position, where at least the portion of the moving tab is drawn outward from the locking body, and wherein the moving tab contacts the locking body at the locking position to deflect the locking body to be engaged with at least the part of the operation portion, and wherein the locking body is disengaged from the operation portion when the moving tab moves to the removal position.

To accomplish the purpose of the present disclosure described above, an applicator assembly may comprise: an applicator body; a medical device including an insertion portion configured to be at least partially insertable into skin of a user and detachably coupled to the applicator body; an insertion unit installed at the applicator body to insert the insertion portion into the skin of the user; an operation portion installed at the applicator body so that at least a part of the operation portion is movable from a deactivated position to an activated position operating the insertion unit; and a backstop portion configured to lock at least the of the operation portion in the activated position to prevent at least the part of the operation portion from returning from the activated position to the deactivated position.

### Advantageous Effects of Invention

According to the present disclosure, by manufacturing the medical device in an assembled state within the applicator, the additional work of a user to attach the medical device to the skin of the user can be minimized, and the medical device can be attached to the skin of the user simply by operating the applicator.

In addition, according to the present disclosure, since at least a part of the operating portion which is operated by the user moves to the activation position that operates the insertion unit and then is locked in the activation position, it is difficult to reuse the applicator after it has been used once, and safe disposal of the applicator is possible.

### BRIEF DESCRIPTION OF THE DRAWINGS

FIG. 1 is a perspective view showing an applicator assembly according to an embodiment of the present disclosure.
FIG. 2 shows the medical device of an applicator assembly according to an embodiment of the present disclosure attached to the skin of a user.
FIG. 3 is a cross-sectional view showing a sensor unit and a base unit of a medical device before they are coupled.
FIG. 4 is a cross-sectional view showing a sensor unit and a base unit coupled.
FIGS. 5 and 6 are exploded perspective views showing a sensor unit of a medical device.
FIG. 7 shows a needle coupled to a sensor unit.
FIG. 8 shows a base unit of a medical device.
FIG. 9 is a cross-sectional view taken along line I-I in FIG. 1.
FIG. 10 is a cross-sectional view taken along line II-II in FIG. 1.
FIG. 11 is an exploded perspective view of an applicator assembly according to an embodiment of the present disclosure.
FIG. 12 shows a locking hook and a moving tab of an applicator separated from a base frame.
FIG. 13 is a perspective view showing a middle frame of an applicator.
FIG. 14 is a perspective view showing the bottom of a middle frame.
FIG. 15 is an exploded view of an insertion unit assembly of an applicator.
FIG. 16 is an exploded perspective view showing a portion of an insertion unit assembly.
FIG. 17 is a perspective view showing an operating portion of an applicator.
FIGS. 18 and 19 show an operating portion located in a deactivated position.
FIGS. 20 and 21 show an operating portion located in an activated position.
FIGS. 22 to 28 show the process of attaching a medical device to the skin of a user using an applicator.

### DETAILED DESCRIPTION OF EMBODIMENTS

Hereinafter, the applicator and applicator assembly for a medical device according to the present disclosure will be described in detail with reference to the drawings.

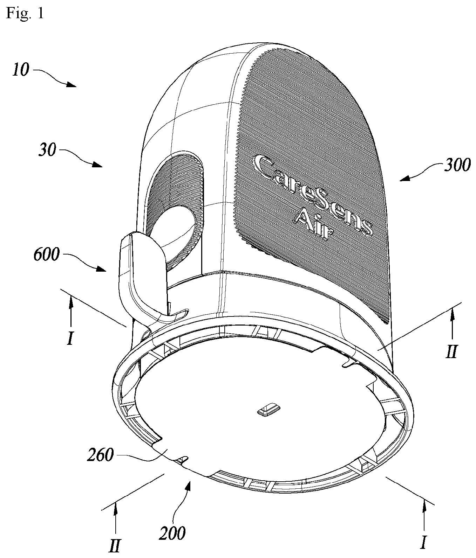

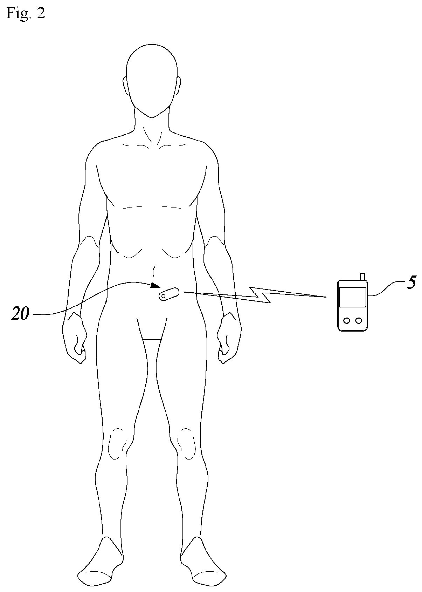

FIG. 1 is a perspective view showing an applicator assembly according to an embodiment of the present disclosure, and FIG. 2 shows a medical device of the applicator assembly according to an embodiment of the present disclosure attached to the skin of a user.

The applicator assembly (10) according to an embodiment of the present disclosure includes a sensor unit (100) having an insertion portion (116) that is inserted into the skin of a user, a base unit (200) that forms the medical device (20) with the sensor unit (100), and an applicator (30) for attaching the medical device (20) to the skin of a user. The applicator assembly (10) may be provided to a user with the sensor unit (100) and the base unit (200) mounted separately on the applicator (30). The sensor unit (100) and the base unit (200) may be coupled to each other by the applicator (30) and attached to the skin of a user.

As shown in FIG. 2, the medical device (20) can be attached to the skin of a user, measure biometric information, and wirelessly transmit the measured data to the external terminal (5). The external terminal (5) may be a variety of devices capable of receiving measurement data from the medical device (20), such as a portable terminal, a medical device, a PC, or a server. Biometric information that the medical device (20) can measure is not limited to specific information. As an exemplary embodiment, the medical device (20) may periodically measure blood glucose level and transmit the blood glucose measurement information to the external terminal (5).

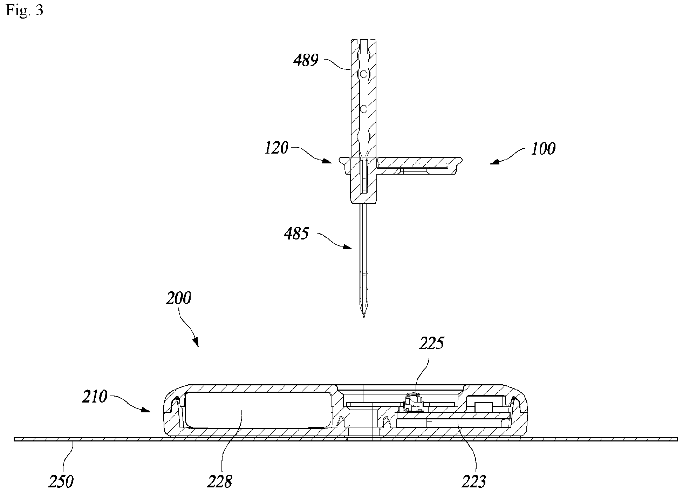

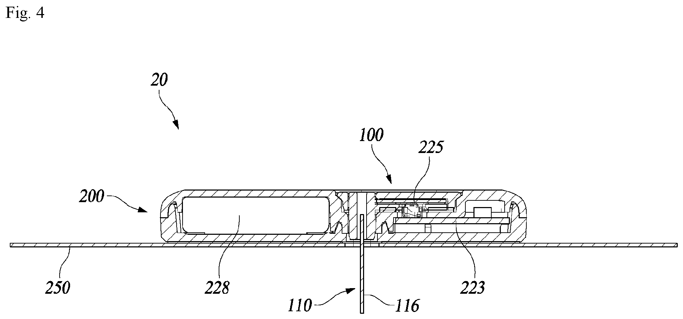

As shown in FIGS. 3 and 4, the medical device (20) includes the sensor unit (100) for measuring the biometric information, and the base unit (200) that is attached to the skin of a user and coupled to the sensor unit (100). The base unit (200) may have electronic components installed therein. This base unit (200) is electrically connected to the sensor unit (100) and can process biometric information measured by the sensor unit (100) and transmit it to the external terminal (5).

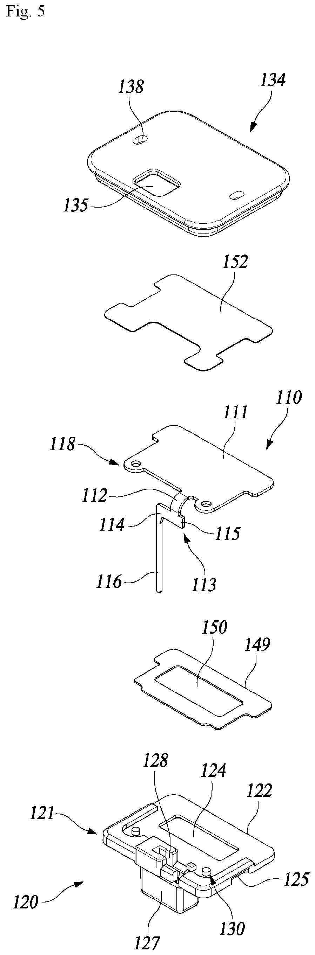

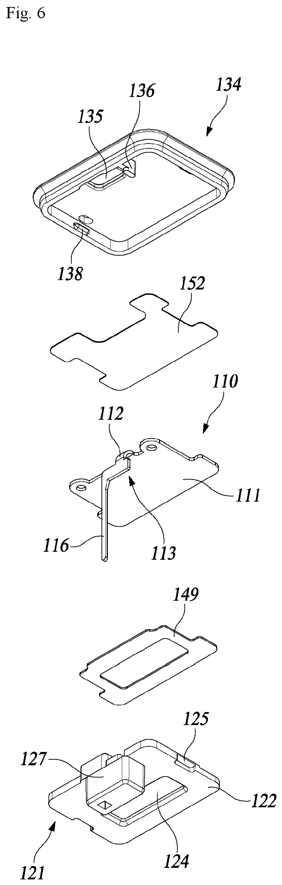

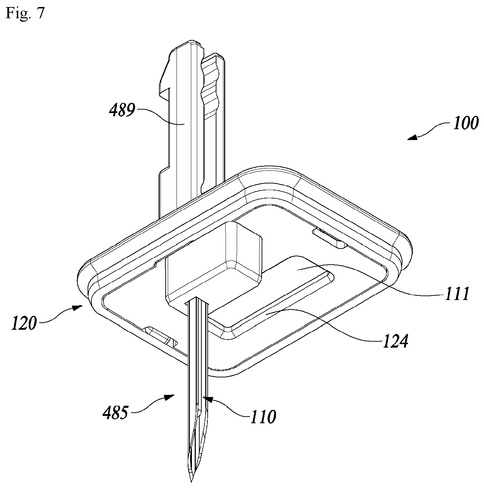

As shown in FIGS. 3 to 7, the sensor unit (100) includes the sensor (110) having an insertion portion (116) that is inserted into the skin of a user, a sensor unit housing (120) to which the sensor (110) is coupled, a sensor adhesive portion (149) and an adhesive pad (152) for fixing the sensor (110) to the sensor unit housing (120).

At least a portion of the sensor (110) may be inserted into the skin of a user to detect an analyte in the skin and generate an electrical signal. The sensor (110) includes a sensor body (111), a connection portion (112) connected to one side of the sensor body (111), a middle portion (113) connected to the sensor body (111) through a connection portion (112), and an insertion portion (116) connected to the middle portion (113) to be inserted into the skin of a user.

The sensor body (111) is disposed inside the sensor unit housing (120). The sensor body (111) is provided with a sensor coupling portion (118). The connection portion (112) extends from one side of the sensor body (111) and connects the sensor body (111) and the middle portion (113) to lie on different planes. After the sensor (110) is manufactured in a flat form in which the sensor body (111) lies on the same plane as the middle portion (113) and the insertion portion (116), the connection portion (112) may be bent and deformed so that the sensor body (111) is manufactured to lie on a different plane from the middle portion (113) and the insertion portion (116).

The middle portion (113) and the insertion portion (116) are connected to the sensor body (111) so as to lie on a different plane from the sensor body (111). In the drawing, the middle portion (113) and the insertion portion (116) are shown to be disposed on the same plane, and the middle portion (113) is disposed perpendicular to the sensor body (111), but the angle between the middle portion (113) and the sensor body (111) or the angle between the insertion portion (116) and the sensor body (111) can be changed in various ways. The middle portion (113) includes a first extension portion (114) protruding from the connection portion (112) in one direction, and a second extension portion (115) protruding from the connection portion (112) in a direction opposite to the direction in which the first extension portion (114) protrudes.

The insertion portion (116) may be connected to the first extension portion (114) and inserted into the skin of a user. The insertion portion (116) is shaped like a relatively thin and long probe so that it can be smoothly inserted into the skin of a user. The connection portion (112), the middle portion (113), and the insertion portion (116) may be provided with a conductive trace electrically connected to the electrode of the sensor body (111). The sensor (110) may be coupled to the sensor unit housing (120) so that the sensor body (111), the connection portion (112), and the middle portion (113) are located inside the sensor unit housing (120), and only the insertion portion (116) protrudes from the sensor unit housing (120).

The sensor unit housing (120) includes a housing base (121) and a housing cap (134) that covers the upper part of the housing base (121). Inside the sensor unit housing (120), a space is provided to accommodate the sensor (110).

The housing base (121) includes a base portion (122) supporting the sensor body (111) and a boss (127) protruding from the surface of the base portion (122). A through hole (124) is formed on one side of the base portion (122) so that the base portion (122) extends in the thickness direction. A detent groove (125) is provided at the edge of the base portion (122). The boss (127) is provided with a housing base hole (128). The insertion portion (116) and a needle (485) for inserting the insertion portion (116) into the skin of a user may be inserted into the housing base hole (128). One surface of the housing base (121) is provided with a housing coupling portion (130) that can couple with the sensor coupling portion (118) of the sensor (110). By coupling the sensor coupling portion (118) with the housing coupling portion (130), the sensor (110) can be stably coupled to the housing base (121). The drawing shows that the sensor coupling portion (118) includes a hole and the housing coupling portion (130) includes a protrusion that can be inserted into the hole, but the shape of the sensor coupling portion (118) or the shape of the housing coupling portion (130) can be changed in various ways.

The housing cap (134) is coupled to the housing base (121) and covers the sensor body (111) placed on the housing base (121) and the upper part of the housing base (121). A housing cap hole (135) is formed in the middle of the housing cap (134) to penetrate the housing cap (134) in the thickness direction. The housing cap hole (135) is connected to the housing base hole (128) of the housing base (121). The needle

(485) may pass through the housing cap hole (135) and the housing base hole (128) and protrude to the outside of the sensor unit housing (120). A sensor unit groove (138) is formed on the upper surface of the housing cap (134). Additionally, the housing cap (134) is provided with a detent protrusion (136) that is inserted into the detent groove (125) of the housing base (121). A retention protrusion (136) is provided on the inner surface of the housing cap (134) which faces the housing base (121).

The retention protrusion (136) is arranged so that its end faces the second extension (115) of the middle portion (113) inserted into the housing base hole (128) to restrict the movement of the middle portion (113) of the sensor (110) so that the middle portion (113) does not lift off. Due to the action of these retention protrusions (136), the insertion portion (116) of the sensor (110) can be stably maintained in a state of protruding from the sensor unit housing (120) by a certain length. And, due to the action of the retention protrusion (136), the insertion portion (116) of the sensor (110) does not retreat from the skin of a user while being inserted into the skin of a user, and can remain inserted at a certain depth into the skin of a user.

The sensor adhesive portion (149) is disposed between the housing base (121) and the sensor body (111) to attach the sensor body (111) to the housing base (121). The sensor adhesive portion (149) may be made in the form of a double-sided tape with adhesive properties on both sides. A sensor adhesive opening (150) is formed in the middle of the sensor adhesive portion (149) to penetrate the sensor adhesive portion (149) in the thickness direction.

The adhesive pad (152) is disposed between the housing cap (134) and the sensor body (111) to attach the sensor body (111) to the housing cap (134). The adhesive pad (152) may be in the form of a double-sided tape with adhesive properties on both sides.

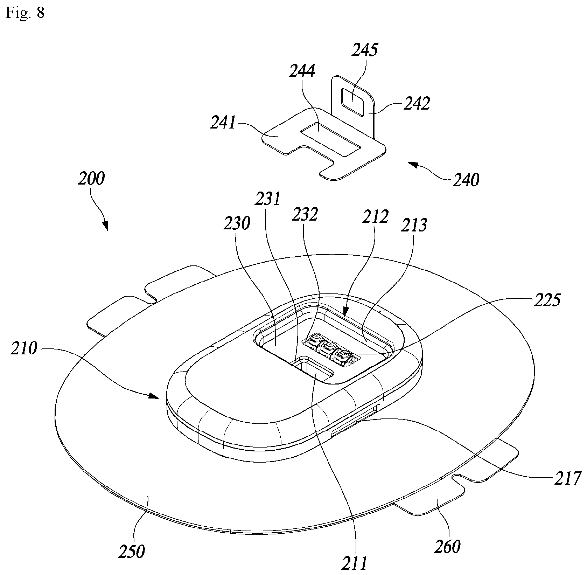

Referring to FIGS. 3, 4, and 8, the base unit (200) includes a base unit housing (210) to which the sensor unit (100) is coupled, electronic components installed inside the base unit housing (210), an adhesive layer (230) provided on the base unit housing (210) to be attached to the sensor unit (100), and a protective sheet (240) covering the adhesive layer (230). The electronic component may include a circuit board (223), an electrical contact portion (225) in contact with the sensor (110), a battery (228), etc. The circuit board (223) may be equipped with a processor chip for processing signals, a communication chip for wireless communication with the external terminal (5), etc.

The base unit housing (210) is provided with an insertion hole (211) through which the insertion portion (116) of the sensor (110) and the needle (485) can pass, and a mounting portion (212) in which the sensor unit housing (120) is coupled. The mounting portion (212) includes a base unit recess (213). The insertion hole (211) is shaped so that the boss (127) of the sensor unit (100) can be fitted and is disposed inside the base unit recess (213). The sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). Some part of a locking hook (360) provided on the applicator (30) may be inserted into the housing groove (217).

Electrical contact portion (225) contacts sensor (110) when sensor unit (100) is coupled to base unit (200). The electrical contact portion (225) is electrically connected to the circuit board (223), and its upper end protrudes higher than the adhesive layer (230). When the sensor unit (100) is coupled to the base unit (200), the electrical contact portion (225) may pass through the through hole (124) and the sensor adhesive opening (150) of the sensor unit (100) and contact the sensor body (111). The electrical contact portion (225) may be elastically deformed when in contact with the sensor body (111) so as to stably contact the sensor body (111).

The adhesive layer (230) is disposed on one side of the base unit housing (210). The adhesive layer (230) may be in the form of a double-sided tape with adhesive properties on both sides. The adhesive layer (230) may attach the sensor unit housing (120) to the base unit housing (210). Adhesion layer holes (231 and 232) are formed in the middle portion of the adhesive layer (230) so as to penetrate the adhesive layer (230) in the thickness direction. One adhesive layer hole (231) is connected to the insertion hole (211). The electrical contact portion (225) may protrude onto the adhesive layer (230) through another adhesive layer hole (232). When the sensor unit (100) is coupled to the base unit (200), the adhesive layer (230) couples the sensor unit housing (120) and the base unit housing (210) and may seal between the sensor unit housing (120) and the base unit housing (210) to prevent moisture or foreign substances from flowing into the electrical contact portion (225).

The protective sheet (240) covers and protects the adhesive layer (230). The protective sheet (240) is made of a material that is detachably attached to the adhesive layer (230). If the adhesive layer (230) is left exposed to the air for a long time, the adhesiveness of the adhesive layer (230) may deteriorate. The protective sheet (240) covers the adhesive layer (230) to prevent the problem of poor adhesiveness of the adhesive layer (230), and it facilitates handling of the base unit (200) by an operator during the manufacturing process of the base unit (200) or the process of assembling the base unit (200) to the applicator (30). The protective sheet (240) includes a protection portion (241) covering the adhesive layer (230) and a wing portion (242) extending from the protection portion (241). A protective sheet opening (244) is formed in the protection portion (241) to penetrate the protection portion (241) in the thickness direction. When the protective portion (241) covers the adhesive layer (230), the protective sheet opening (244) may be connected to the adhesive layer hole (232) of the adhesive layer (230). A protective sheet hole (245) is formed in the wing portion (242) to penetrate the wing portion (242) in the thickness direction. The wing portion (242) may extend outward from the base unit housing (210) and be coupled to the moving tab (600) of the applicator (30).

The shape of the protective sheet (240) is not limited to that shown and can be changed in various ways.

The base unit (200) is mounted on the applicator (30) with the protective sheet (240) attached to the adhesive layer (230). The protective sheet (240) may be separated from the adhesive layer (230) by the moving tab (600) before the sensor unit (100) is coupled to the base unit (200). After the protective sheet (240) is removed from the base unit (200) by the moving tab (600), the sensor unit (100) may move toward the base unit (200) and be coupled to the base unit (200).

An adhesive portion (250) is provided on the surface of the base unit housing (210). The adhesive portion (250) may adhere the base unit housing (210) to the skin of a user. A hole through which the insertion portion (116) of the sensor (110) and the needle (485) can pass may be provided in the middle of the adhesive portion (250). The adhesive portion (250) may be covered and protected with a protective sheet (260). The protective sheet (260) may be removed during the process of attaching the base unit (200) to the skin of a user.

The sensor unit (100) and the base unit (200) are installed in the applicator (30) in a separated state, and are coupled to each other in the process of operating the applicator (30) to insert the sensor (110) into the skin of a user, thereby creating a medical device (20). The sensor unit (100) may be mounted on the applicator (30) while the needle (485) coupled, and moved toward the base unit (200) with the needle (485) coupled thereto to be coupled to the base unit (200). After the sensor unit (100) is coupled to the base unit (200), the needle (485) is separated from the sensor unit (100), and only the medical device (20) remains on the skin of a user.

As another exemplary embodiment, the base unit (200) may be configured to simply support the sensor unit (100) without being separated from the skin of a user without electronic components. In this case, a separate electronic unit that can process biometric information measured by the sensor unit (100) and transmit it to the external terminal (5) may be detachably coupled to the base unit (200). A separate electronic unit may be coupled to the base unit (200) to be electrically connected to the sensor unit (100) after the sensor unit (100) is coupled to the base unit (200).

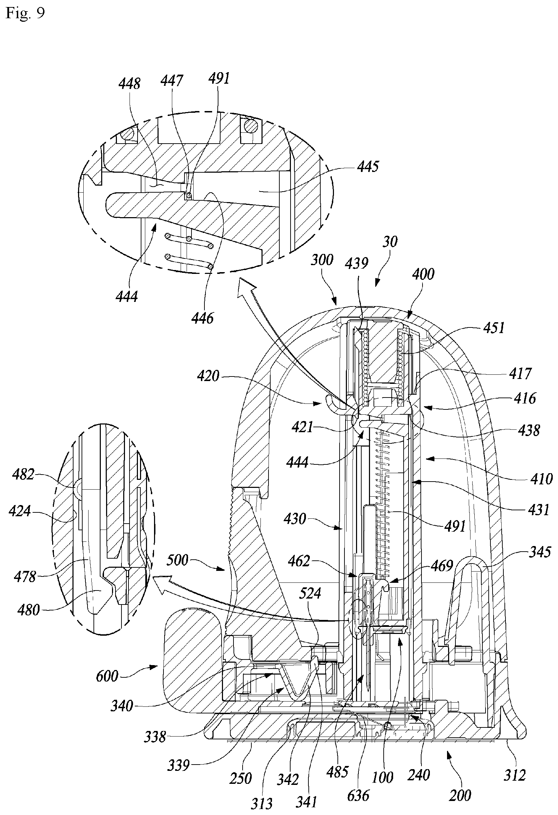

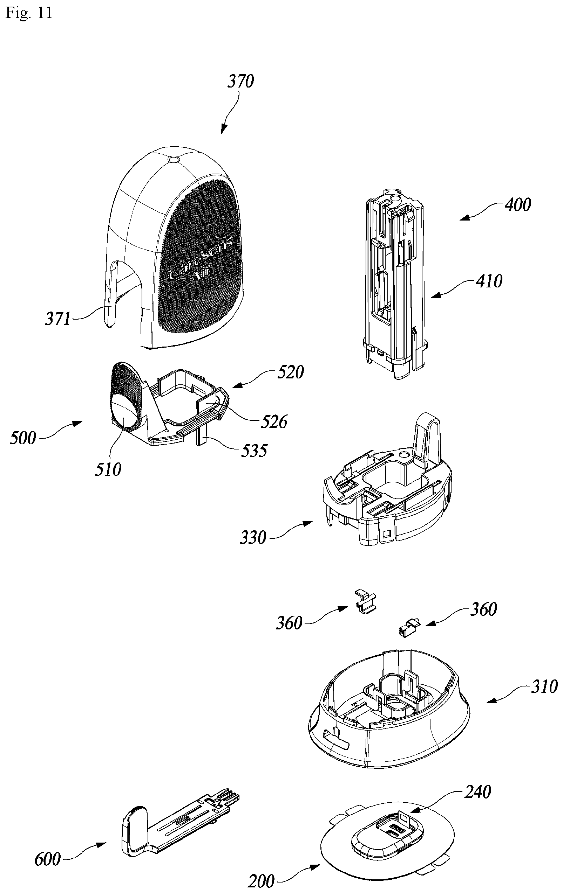

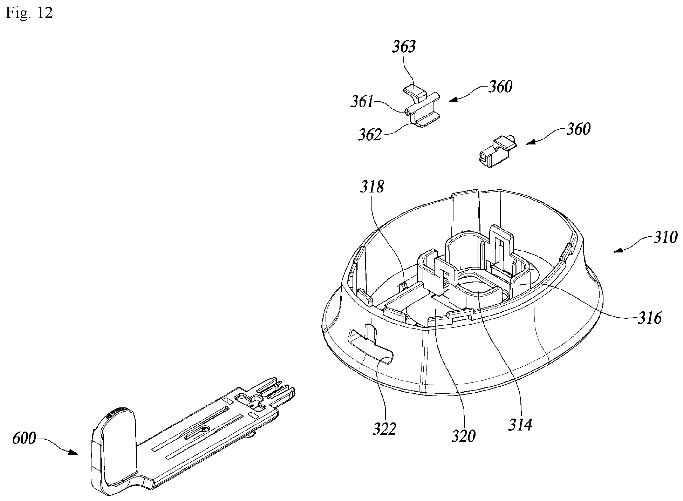

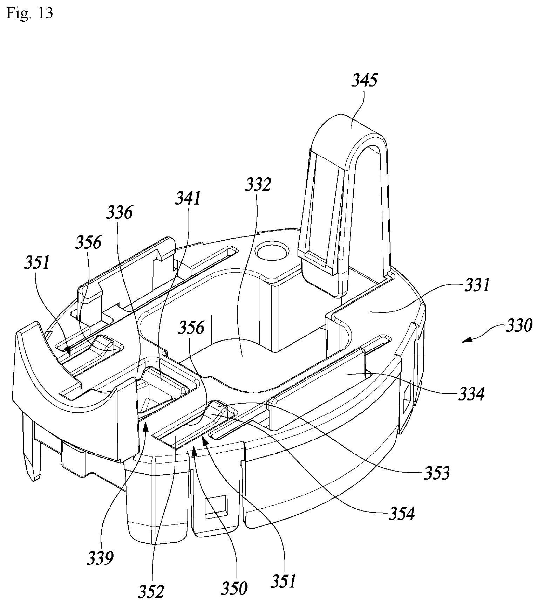

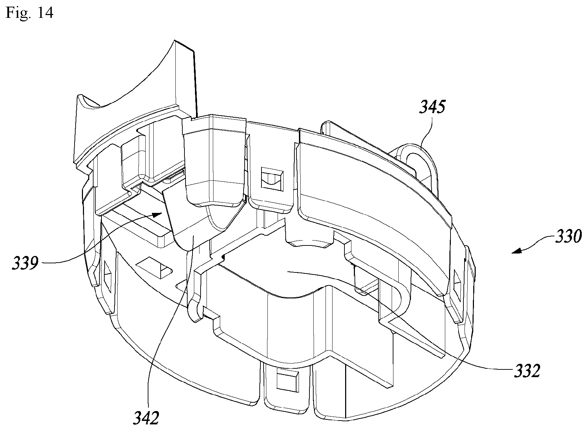

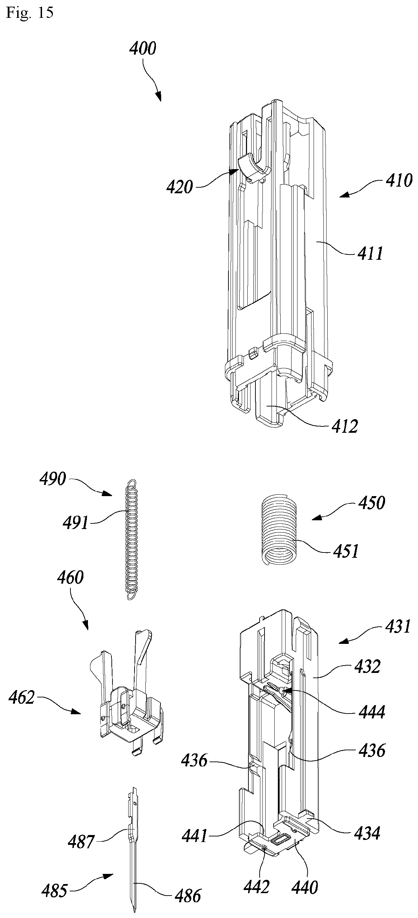

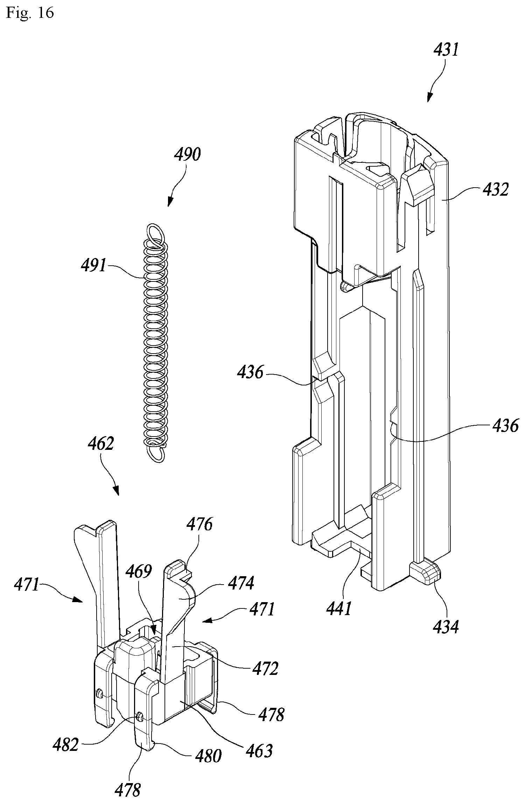

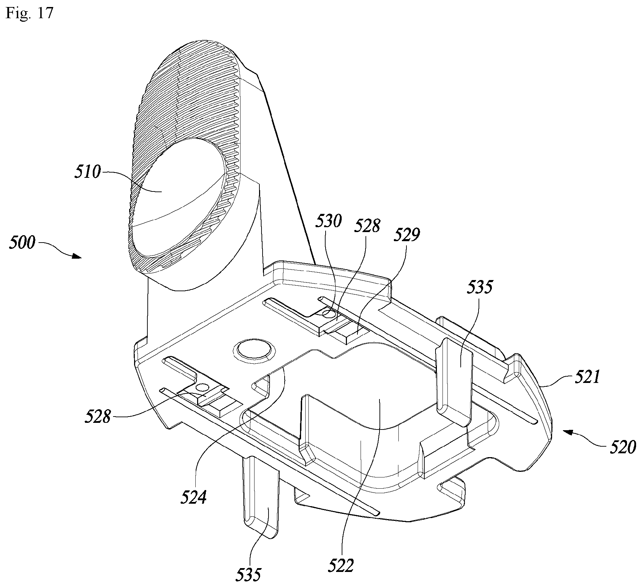

Referring to FIGS. 9 to 21, the applicator (30) may operate in a state in which the sensor unit (100) and the base unit (200) are mounted to couple the sensor unit (100) and the base unit (200), and attach the medical device (20), in which the sensor unit (100) and the base unit (200) are coupled, to the skin of a user. The applicator (30) is separated from the medical device (20) after attaching the medical device (20) to the skin of a user, and the medical device (20) remains attached to the skin by the adhesive portion (250).

The applicator (30) includes an applicator body (300) to which the base unit (200) is detachably coupled, an insertion unit assembly (400) that includes an insertion unit (430) for inserting the sensor (110) into the skin of a user, an operating portion (500) installed on the applicator body (300) so that the user can operate, and a moving tab (600) for removing the protective sheet (240). The insertion unit (430) may move the sensor unit (100) from a first position spaced apart from the base unit (200) by a preset distance to a second position coupled with the base unit (200).

The applicator body (300) includes a base frame (310), a middle frame (330) disposed on the base frame (310), and a top case (370) that is coupled to the middle frame (330) and covers the middle frame (330).

The base frame (310) has a bottom portion (312) that can be in contact with the skin of a user. The bottom portion (312) of the base frame (310) is provided with a recess (313) on which the base unit (200) is mounted. At least a portion of the base unit (200) may be accommodated in the recess (313) so that the adhesive portion (250) can face the skin of a user. A base frame opening (314) is formed in the middle of the base frame (310) to penetrate the base frame (310) in the thickness direction. A fence portion (316) is provided around the base frame opening (314). The fence portion (316) may support the insertion unit assembly (400). Support portions (318) are provided on both sides of the base frame opening (314). The support portion (318) may rotatably support the locking hook (360) for detachably coupling the base unit (200) to the applicator body (300).

The locking hook (360) may engage the base unit (200) mounted on the recess (313) to keep the base unit (200) placed in the recess (313). The locking hook (360) may be disengaged from the base unit (200) after the medical device (20) is attached to the skin of a user. The locking hook (360) is pivotally coupled to the support portion (318). The locking hook (360) includes a pivot portion (361) rotatably coupled to the support portion (318), a hook portion (362) protruding from one side of the pivot portion (361), and an extension portion (363) protruding from the other side of the pivot portion (361). The hook portion (362) may be engaged with the base unit (200) by inserting its end into the housing groove (217) of the base unit (200). The end of the hook portion (362) may be located inside of the recess (313) of the base frame (310) and engaged with the base unit (200). The locking hook (360) may keep the base unit (200) coupled to the applicator body (300) by restricting the movement of the hook portion (362) in a state in which the hook portion (362) is engaged with the base unit (200). After the sensor unit (100) is coupled to the base unit (200), the locking hook (360) may be released from its restraint by the operating portion (500) and disengaged from the base unit (200).

A stage (320) is provided on an inner side of the base frame (310). The stage (320) supports the moving tab (600) so that the moving tab (600) can move linearly stably. An inclined portion (321) is provided at one end of the stage (320).

In addition, the base frame (310) is provided with an entrance (322). The entrance (322) is formed on one side of the outer surface of the base frame (310). The moving tab (600) placed on the stage (320) may be pulled out to the outside of the base frame (310) through the entrance (322).

The specific configuration of the base frame (310) is not limited to that shown and can be changed in various ways.

The middle frame (330) includes a stage (331) that supports the operating portion (500). A middle frame opening (332) connected to the base frame opening (314) of the base frame (310) is provided in the middle of the stage (331). A column member (410) of the insertion unit assembly (400) may be inserted into base frame opening (314) through middle frame opening (332). Guide protrusions (334) are provided on both edges of the stage (331). The guide protrusion (334) may guide the operating portion (500) to linearly move from the deactivated position to the activated position. Here, the deactivation position is a position in which the operating portion (500) does not operate the insertion unit (430) so that the sensor unit (100) waits in the first position, and the activation position is a position in which the operating portion (500) may operate the insertion unit (430) so that the sensor unit (100) may move to the second position.

The middle frame (330) is provided with a locking unit (338) to restrict the movement of the operating portion (500) so that the operating portion (500) does not move from the deactivated position, an elastic pressing portion (345) to elastically support the operating portion (500), and a backstop member (351) constituting the backstop portion (350) for restricting the movement of the operating portion (500). The backstop portion (350) may lock the operating portion (500), which has moved to the activated position, to the activated position so that it does not return to the deactivated position.

The locking unit (338) is disposed of inside the through hole (336) formed in the middle of the stage (331). The locking unit (338) includes a locking body (339) connected to the middle frame (330) so as to be engaged with or disengaged from the operating portion (500). The locking body (339) includes a body portion (340) elastically and deformably connected to the middle frame (330), a hook portion (341) connected to one side of the body portion (340) so as to be engaged with the operating portion (500), and a leg portion (342) connected to the other side of the body portion (340) so as to contact the moving tab (600). The locking body (339) may be deflected in a direction in which the hook portion (341) engages with the operating portion (500) when the leg portion (342) contacts the moving tab (600). In addition, the locking body (339) may be deflected in a direction in which the hook portion (341) is disengaged from the operating portion (500) when the leg portion (342) leaves the moving tab (600). That is, the locking body (339) can maintain a position in which the hook portion (341) is not engaged with the operating portion (500) when no other external force is applied. And the locking body (339) is elastically deformed so that the hook portion (341) engages with the operating portion (500) when the moving tab (600) contacts the leg portion (342).

The configuration of the locking unit (338) is not limited to that shown and may be changed in various ways.

The elastic pressing portion (345) is in contact with one side of the operating portion (500) and can elastically support the operating portion (500) to prevent the operating portion (500) from easily moving from the deactivated position to the activated position. That is, the elastic pressing portion (345) may generate a resistance force that resists the force that a user pushes the operating portion (500). The elastic pressing portion (345) can prevent accidental movement of the operating portion (500) by allowing it to move when the operating portion (500) receives a pressing force greater than a preset force.

The backstop member (351) is disposed in the recess (356) of the middle frame (330) and locks the operating portion (500) in the activated position to prevent the operating portion (500), which has moved to the activated position, from returning to the deactivated position. The backstop member (351) includes a backstop member body (352) elastically and deformably connected to the middle frame (330) and a backstop member snap (353) provided on one side of the backstop member body (352) so as to be engaged with one side of the operating portion (500). The backstop member snap (353) may protrude above the stage (331). An inclined surface (354) is provided on one side of the backstop member snap (353). The backstop member (351) may lock the operating portion (500) in the activation position by coupling the backstop member snap (353) with one side of the operating portion (500) when the operating portion (500) moves to the activation position.

The top case (370) covers the upper part of the middle frame (330). One side of the top case (370) is provided with a top case opening (371) where the operating portion (500) is disposed.

In addition to the configuration including the base frame (310), the middle frame (330), and the top case (370) as shown, the applicator body (300) may be changed to various other configurations which may be coupled to the insertion unit assembly (400) to support the insertion unit assembly (400) and to which the base unit (200) can be detachably coupled. Additionally, the applicator body (300) may be coupled to the base unit (200) in various other ways other than using the tilting type locking hook (360).

The insertion unit assembly (400) includes a column member (410) which is fixed to the applicator body (300) and an insertion unit (430) which is supported on the column member (410) to be coupled to the sensor unit (100) and moves the sensor unit (100) from the first position to the second position. The insertion unit (430) includes a shuttle (431) movably installed inside the column member (410) and a needle assembly (460) that can move together with the shuttle (431). The shuttle (431) is coupled to the sensor unit (100) and can move from the first position to the second position together with the sensor unit (100). The needle assembly (460) includes a carrier (462) that can relatively move with respect to the shuttle (431) and a needle (485) that is coupled to the carrier (462) and can be inserted into the skin of a user.

The column member (410) is fixed to the applicator body (300) and supports the insertion unit (430). The column member (410) includes a column member body (411) having a column member opening (412) opened outward at one end. A space is provided inside the column member body (411) to accommodate the insertion unit (430). A release portion (414) is provided on the inner surface of the column member body (411). The release portion (414) is a part of the carrier (462) that can be contacted while the carrier (462) moves toward the base unit (200). Due to the action of the release portion (414), the carrier (462) is decoupled from the shuttle (431) and can relatively move with respect to the shuttle (431).

A clamping portion (416) and a blocking portion (420) are provided on both sides of the column member body (411) to restrict the movement of the shuttle (431).

The clamping portion (416) has a clamping portion detent (417) that can be engaged with one side of the shuttle (431). The insertion unit assembly (400) may be coupled to the applicator body (300) with the shuttle (431) temporarily fixed to the column member (410) by the clamping portion (416). When the clamping portion detent (417) of the clamping portion (416) engages with the shuttle latch (438) of the shuttle (431), the shuttle 431 is temporarily fixed to the column member (410) and at this time, the shuttle (431) cannot move. By temporarily fixing the shuttle (431) to the column member (410) using the clamping portion (416), it is possible to prevent the shuttle (431) from accidentally moving during the assembly process of the insertion unit assembly (400). After the insertion unit assembly (400) is coupled to the applicator body (300) during the manufacturing process of the applicator assembly (10), the clamping portion (416) may be disengaged from the shuttle (431). As shown in FIG. 9, the applicator assembly (10) may be provided to a user with the clamping portion (416) disengaged from the shuttle (431).

The blocking portion (420) may be used to fix the shuttle (431), which has reached the second position, in the second position. The blocking portion (420) has a blocking portion detent (421) engaged with one side of the shuttle (431). The blocking portion (420) may prevent the shuttle (431) from returning to the first position from the second position by coupling a detent portion (439) of the shuttle (431) after the shuttle (431) moves to the second position. The blocking portion (420) may prevent the shuttle (431) from being reloaded or the applicator (30) from being reused by restricting the movement of the shuttle (431).

The shuttle (431) is installed to be linearly movable inside the column member (410). The shuttle (431) includes a shuttle body (432) with a space inside to accommodate the needle assembly (460). A shuttle protrusion (434) is provided on one side of the shuttle body (432). The shuttle protrusion (434) protrudes from the shuttle body (432) so as to come into contact with the operating portion (500). The shuttle (431) is fixed in the first position when the shuttle protrusion (434) comes into contact with the operating portion (500), and can move to the second position when the shuttle protrusion (434) leaves the operating portion (500). A shuttle detent (436) with which a carrier latch (476) of the carrier (462) can be engaged is provided inside the shuttle body (432). With the carrier latch (476) engaged with the shuttle detent (436), the carrier (462) moves with the shuttle (431) and cannot relatively move with respect to the shuttle (431). And when the carrier latch (476) is disengaged from the shuttle detent (436), the carrier (462) can relatively move with respect to the shuttle (431). A shuttle bottom portion (440) in contact with the sensor unit (100) is provided at one end of the shuttle body (432). The shuttle bottom portion (440) is provided with a shuttle through hole (441) and a pressing protrusion (442). The needle (485) may protrude out of the shuttle body (432) through the shuttle through hole (441). The pressing protrusion (442) is provided to be inserted into the sensor unit groove (138) of the sensor unit (100). When the sensor unit (100) is mounted on the insertion unit (430), the pressing protrusion (442) is inserted into the sensor unit groove (138) of the sensor unit (100), so that the sensor unit (100) can maintain its fixed position without moving. A fixing portion (444) is provided inside the shuttle (431). The fixing portion (444) is for fixing the needle release driver (490) that provides moving force to the carrier (462). The fixing portion (444) includes a fixing portion groove (445), a contact surface (446), a barrier portion (447), and an opening (448). The contact surface (446) is disposed in the fixing portion groove (445), and the opening (448) is disposed relatively outside the fixing portion groove (445) and connected to the fixing portion groove (445). The barrier portion (447) is disposed between the fixing portion groove (445) and the opening (448) and protrudes from the contact surface (446).

The shuttle (431) moves by receiving movement force from the shuttle driver (450). The shuttle driver (450) includes a forward elastic member (451) that applies elastic force to the shuttle (431) in the direction of moving from the first position to the second position. The forward elastic member (451) has one end in contact with one side of the column member (410) and the other end in contact with one side of the shuttle (431). The shuttle (431) may be installed inside the column member (410) with the forward elastic member (451) elastically deformed. The shuttle (431) may stand by at the first position in a state in which the forward elastic member (451) is elastically deformed by the shuttle protrusion (434) coming into contact with the operating portion (500). And when the operating portion (500) moves and the shuttle protrusion (434) deviates from the operating portion (500), the shuttle (431) may move to the second position by the elastic force of the forward elastic member (451).

The shuttle driver (450) may be changed to various other configurations that can provide a moving force to the shuttle (431) in addition to the configuration including the forward elastic member (451) with a coil spring.

The needle assembly (460) includes a carrier (462) to which the sensor unit (100) is detachably coupled, and a needle (485) coupled to the carrier (462) to penetrate the sensor unit (100). The needle (485) may be coupled to the sensor unit (100) and moves forward from the first position to the second position with the sensor unit (100) to be inserted into the skin of a user. Additionally, the needle (485) may retract together with the carrier (462) in the second position and then may be separated from the skin of a user and the sensor unit (100).

The carrier (462) is coupled to the shuttle (431) to be able to move relatively. The carrier (462) may move forward from the first position to the second position together with the shuttle (431) by partially coupling the shuttle (431). Then, the carrier (462) is decoupled from the shuttle (431) in the second position and may retreat in a direction away from the sensor unit (100). The carrier (462) includes a carrier body (463) to which the needle 485 is coupled, a plurality of carrier wings (471) extending from the carrier body (463) so as to be elastically deformed, and a plurality of grip arms (478) connected to the sides of the carrier body (463). The carrier body (463) is provided with a fixing portion (469) to which the needle release driver (490) is coupled. The carrier wing (471) includes a wing body (472) elastically and deformably connected to the carrier body (463), and a trigger (474) and a carrier latch (476) which are protruding from the wing body (472). The trigger (474) may contact the release portion (414) of the column member (410) while the carrier (462) moves from the first position to the second position together with the shuttle (431).

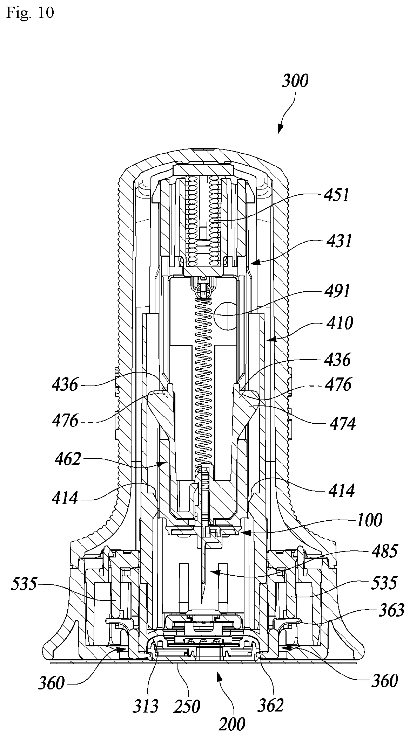

As shown in FIG. 10, the release portion (414) is disposed inside the column member (410) so as to contact the trigger (474). While the carrier (462) is moving from the first position to the second position, the trigger (474) may come into contact with the release portion (414) and the carrier wing (471) may be elastically deformed. The carrier latch (476) protrudes outward from the wing body (472). The carrier latch (476) may engage the shuttle detent (436) of the shuttle (431). In a state in which the carrier latch (476) is engaged with the shuttle detent (436), the carrier (462) can maintain the elastically deformed state of the backward elastic member (491) of the needle release driver (490), cannot move in the direction in which the elastic force of the backward elastic member (491) acts. And when the trigger (474) contacts the release portion (414) and the carrier wing (471) is elastically deformed, the carrier latch (476) may deviate from the shuttle detent (436) and the carrier (462) may move.

The grip arm (478) is provided on the side of the carrier body (463) to be elastically deformable. A plurality of grip arms (478) may be arranged to face each other on both sides of the carrier body (463) and engage with the sensor unit (100). The grip arm (478) is provided with a grip protrusion (480) that engages with the sensor unit housing (120) and a protrusion (482) that contacts the inner wall (424) of the column member (410). The carrier (462) is coupled to the shuttle (431) so that the carrier body (463) is located inside the shuttle (431) and the end of the grip arm (478) protrudes from the shuttle (431). The carrier (462) may fix the sensor unit (100) in such a manner that the grip arm (478) engages with the sensor unit housing (120). At this time, the surface of the sensor unit housing (120) is in close contact with the shuttle bottom portion (440) of the shuttle (431), and the pressing protrusion (442) of the shuttle (431) is inserted into the sensor unit groove (138) of the sensor unit housing (120). Therefore, the sensor unit (100) can remain stably coupled to the shuttle (431).

And the carrier (462) may position the sensor unit (100) in the first position when the insertion unit assembly (400) is coupled to the applicator body (300), as shown in FIG. 9. When the carrier (462) is positioned in the first position, the protrusion (482) of the grip arm (478) contacts the inner wall (424) of the column member (410). Accordingly, the grip arm (478) is deflected in a direction in which it engages the sensor unit housing (120), and the carrier (462) can remain stably coupled to the sensor unit (100).

Meanwhile, when the sensor unit (100) and the carrier (462) move toward the base unit (200) and the sensor unit (100) is coupled with the base unit (200), the protrusion (482) deviates from the inner wall (424) of the column member (410). Therefore, when the sensor unit (100) is coupled to the base unit (200), the fixing force of the grip arm (478) is weakened. Since the sensor unit (100) is fixed to the base unit (200) by the adhesive layer (230), when the carrier (462) is pulled in a direction away from the medical device (20) by the needle release driver (490), the grip arm (478) may be separated from the sensor unit (100).

The carrier (462) can be relatively moved with respect to the shuttle (431) by the needle release driver (490). The needle release driver (490) includes a backward elastic member (491) in the form of a tension spring. The backward elastic member (491) may apply an elastic force to the carrier (462) in a direction away from the sensor unit (100) to move the carrier (462) together with the needle (485) so as to be separated from the sensor unit (100). One end of the backward elastic member (491) is coupled to the fixing portion (444) of the shuttle (431). In the process of assembling the insertion unit (430), one end of the backward elastic member (491) enters the fixing portion groove (445) through the opening (448). One end of the backward elastic member (491) is in close contact with the contact surface (446) by the elastic force of the backward elastic member (491) while entering the fixing portion groove (445). At this time, one end of the backward elastic member (491) is blocked by the barrier portion (447) and cannot escape from the fixing portion (444) by passing through the opening (448). Therefore, the backward elastic member (491) can remain stably coupled to the fixing portion (444). The other end of the backward elastic member (491) is fixed to the fixing portion (469) of the carrier (462). The fixing portion (469) of the carrier (462) may have the same configuration as the fixing portion (444) of the shuttle (431) or may have a different configuration.

The needle release driver (490) can be changed to various other configurations that can provide a moving force to the carrier (462) in addition to the configuration including the backward elastic member (491) in the form of a coil spring.

The needle (485) is fixed to the carrier (462) and can move from the first position to the second position while coupled to the sensor unit (100). The needle (485) has a sharp tip so that it can pierce the skin of a user and be smoothly inserted into the skin. When the sensor unit (100) moves to the second position, the needle (485) penetrates the skin prior to the sensor (110) so that the sensor 110 can be stably inserted into the skin. The needle (485) is separated from the skin of a user after the sensor (110) is inserted into the skin. The needle (485) includes a needle body (486) that can be inserted into the skin of a user, and a needle head (487) coupled to the carrier (462). A needle coupler (489) for fixing the needle head (487) to the carrier (462) is coupled to the needle head (487). The needle coupler (489) may be fixed to the inside of the carrier (462) to fix the needle head (487) to the carrier (462).

The specific configuration of the needle (485) is not limited to that shown and may be changed in various ways. Additionally, the coupling method of the needle (485) and the carrier (462) may also be changed in various ways.

The insertion unit assembly (400) may be coupled to the applicator body 300 in a state in which the forward elastic member (451) of the shuttle driver (450) and the backward elastic member (491) of the needle release driver (490) are elastically deformed. Additionally, the insertion unit assembly (400) may be displaced on the applicator body (300) while being coupled to the sensor unit (100). The applicator assembly (10) can be simply assembled to the applicator body (300) by forming a single assembly in which the shuttle (431) and the needle release driver (490), which constitute the insertion unit (430), together with the column member (410). The insertion unit assembly (400) may be coupled to the applicator body (300) in a state in which the forward elastic member (451) of the shuttle driver (450) for moving the shuttle (431) and a backward elastic member (491) of the needle release driver (490) for moving the needle assembly (460) are elastically deformed. Additionally, the insertion unit assembly (400) may be displaced on the applicator body (300) in a state of being coupled to the sensor unit (100). The applicator assembly (10) according to an embodiment of the present disclosure can be simply assembled to the applicator body (300) by forming a single assembly in which a shuttle (431) constituting the insertion unit (430), a shuttle driver (450), a needle assembly (460), and a needle release driver (490) together with a column member (410).

The operating portion (500) is installed on the applicator body (300) to be operated by the user and move from the inactive position to the active position. The operating portion (500) includes a button portion (510) exposed to the outside of the applicator body (300), a stopper portion (520) disposed on the inside of the applicator body (300) so as to contact the shuttle (431), and a stopper protrusion (535) for restraining the movement of the locking hook (360). The operating portion (500) may be installed on the applicator body (300) to move linearly in a direction intersecting the moving direction of the shuttle (431), more preferably in a direction perpendicular to the moving direction of the shuttle (431).

The stopper portion (520) includes a stopper portion body (521) supported on the stage (331) of the middle frame (330), and a support portion (526) for supporting the shuttle (431). A stopper opening (522) is formed in the middle of the stopper portion body (521) to penetrate the stopper portion body (521) in the thickness direction. A backstop detent (528) with which the backstop member (351) can be engaged is provided on the lower surface of the stopper portion body (521). The backstop detent (528) forms the backstop portion (350) together with the backstop member (351). When the operating portion (500) is moved to the activated position by a user, the backstop detent (528) engages with the backstop member snap (353) of the backstop member (351), so that the operating portion (500) is locked in the activated position and cannot return to the inactivated position. The lower surface of the stopper portion body (521) is provided with an accommodation groove (529) and a detent groove (530) in which the backstop member snap (353) can be accommodated.

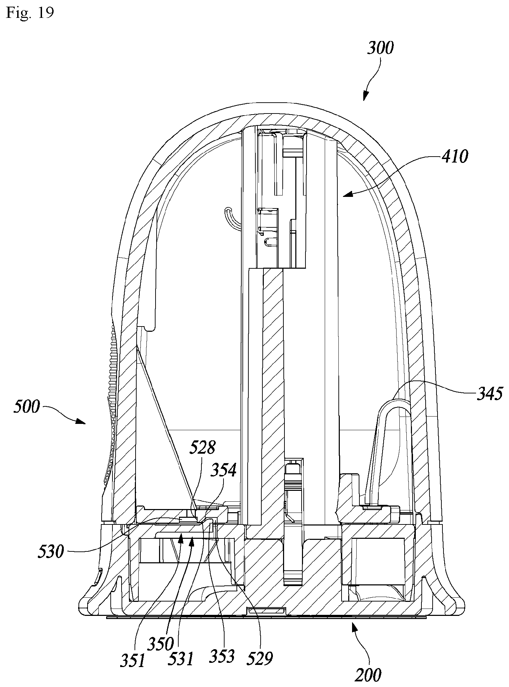

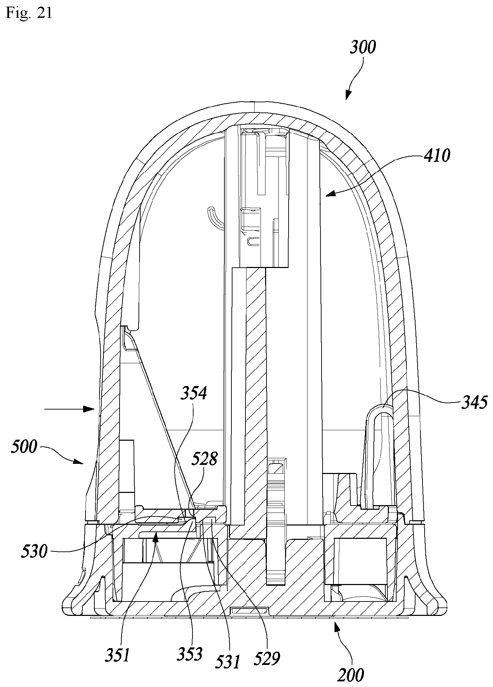

As shown in FIG. 19, the backstop member snap (353) is accommodated in the accommodation groove (529) when the operating portion (500) is located in the deactivated position. And as shown in FIG. 21, when the operating portion (500) is located in the activated position, the backstop member snap (353) is accommodated in the detent groove (530) and engages with the backstop detent (528). An inclined surface (531) is provided between the accommodation groove (529) and the detent groove (530). When the operating portion (500) is pushed from the deactivated position to the activated position by a user, the inclined surface (354) of the backstop member (351) may move along the inclined surface (531) of the stopper portion (520). Therefore, when the operating portion (500) moves, the movement resistance caused by the backstop member (351) can be reduced.

The configuration of the backstop portion (350) for locking the operating portion (500) in the activated position may be changed in various ways. As another exemplary embodiment, a backstop member may be provided on the operating portion and a backstop detent may be disposed on the applicator body.

A stopper detent (524) is provided on one side of the stopper portion body (521). The stopper portion (520) cannot move when the locking body (339) is engaged with the stopper portion detent (524). At this time, even if the button portion (510) is manipulated, the stopper portion (520) cannot move from the deactivated position and may maintain the state of supporting the shuttle (431). The stopper portion (520) may move to the activated position in a state in which the locking body (339) is disengaged from the stopper portion detent (524).

The support portion (526) is disposed at the edge of the stopper portion opening (522). The support portion (526) may protrude from the stopper portion body (521) and support the shuttle protrusion (434) of the shuttle (431). When the stopper portion (520) is located in the deactivated position, the support portion (526) supports the shuttle protrusion (434) to restrict the movement of the shuttle (431) placed in the first position. And when the stopper portion (520) moves to the activated position, the support portion (526) deviates from the shuttle protrusion (434), so that the shuttle (431) can move to the second position.

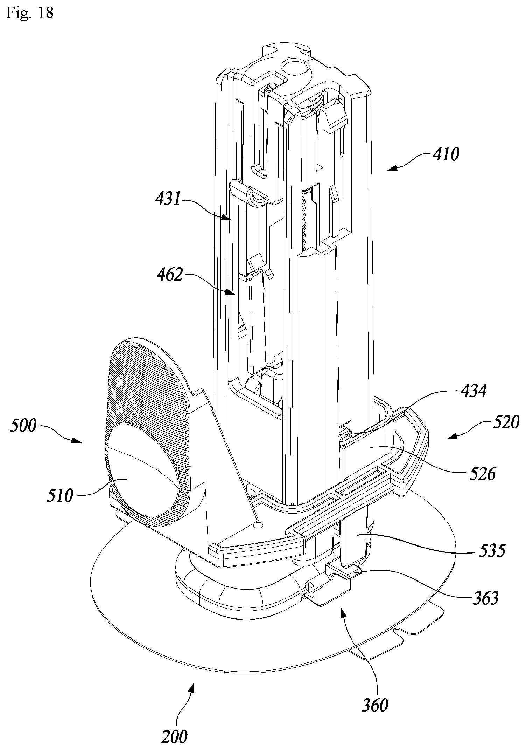

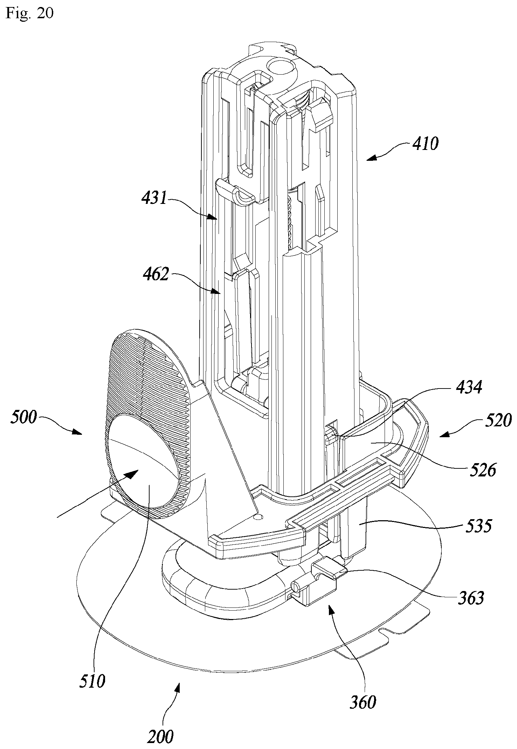

The stopper protrusion (535) protrudes from the stopper portion body (521) so as to contact one side of the locking hook (360). When the operating portion (500) is located in the deactivated position, the stopper protrusion (535) may restrain the movement of the locking hook (360) so that the locking hook (360) does not tilt. On the other hand, when the operating portion (500) is located in the activated position, the stopper protrusion (535) may release the restriction on the locking hook (360). As shown in FIG. 18, when the operating portion (500) is located in the deactivated position, the stopper protrusion (535) is located on the extension portion (363) of the locking hook (360). At this time, since the stopper protrusion (535) may contact the extension portion (363), the locking hook (360) cannot be tilted to disengage from the base unit (200). Therefore, when the operating portion (500) is located in the deactivated position, the base unit (200) can be coupled to the applicator body (300) and maintained in a state in which the locking hook (360) is stably engaged with the base unit (200). Meanwhile, as shown in FIG. 20, when the operating portion (500) is located in the activated position, the stopper protrusion (535) deviates from the extension portion (363). At this time, the locking hook (360) can be tilted without interfering with the stopper protrusion (535). Therefore, when the operating portion (500) is located in the activated position, the locking hook (360) may be tilted to disengage from the base unit (200), and the base unit (200) may be released from the applicator body (300).

The moving tab (600) is coupled to the applicator body (300) so that the protective sheet (240) can be removed by being manipulated by a user. At least a portion of the moving tab (600) may be pulled out from the inside of the applicator body (300) to the outside through an entrance (328) of the applicator body (300). The moving tab (600) is supported on the stage (325) of the applicator body (300) and can move linearly in a direction perpendicular to the direction of movement of the sensor unit (100). The moving tab (600) may move from an initial position coupled with the protective sheet (240) of the base unit (200) located in the second position to a removal position where the protective sheet (240) is separated from the adhesive layer (230). The moving tab (600) includes a pressing portion (636) capable of pressing the protective sheet (240). When the moving tab (600) is located in the initial position, the pressing portion (636) may press the protective sheet (240) toward the adhesive layer (230). Therefore, before the protective sheet (240) is separated from the adhesive layer (230), the protective sheet (240) can keep stably covering the adhesive layer (230). When the moving tab (600) is pulled to the removal position, the pressing portion (636) may be elastically deformed in contact with the inclined portion (321) of the base frame (310).

In addition to the function of removing the protective sheet, the moving tab (600) has a function of restricting the movement of the stopper portion (520) with the locking unit (338). As shown in FIG. 9, when the operating portion (500) is located in the deactivated position and the moving tab (600) is located in the initial position, the moving tab (600) supports the locking body (339) of the locking unit (338). At this time, as the leg portion (342) of the locking body (339) is connected to the moving tab (600), the locking body (339) is deflected in a direction in which the hook portion (341) engages with the stopper portion detent (524) of the operating portion (500). At this time, even if the operating portion (500) is manipulated, the stopper portion (520) cannot move. Meanwhile, when the moving tab (600) is pulled out from the entrance (322), the locking body (339) is disengaged from the operating portion (500) and the operating portion (500) can move.

The configuration of the moving tab (600) is not limited to that shown and can be changed in various ways. For example, the drawing shows that the moving tab (600) completely exits the entrance (328) when moved to the removal position, but the moving tab (600) may be installed so that only a portion comes out through the entrance (328) and the remaining portion is located in the applicator body (300) when moving to the removal position. As another exemplary embodiment, the moving tab may be installed in a push type. In this case, the moving tab is installed on the applicator body (300) to move linearly in a direction intersecting the moving direction of the sensor unit (100), and can be moved by a user to remove the protective sheet (240) or release the retaining force on the operating portion (500). Additionally, the moving tab may be installed to be movable in various directions on the applicator body (300) so that it can be moved by the user.

Additionally, the drawing shows that the locking body (339) is installed on the applicator body (300) to be moved by the moving tab (600), but the moving tab may be configured integrally with the locking body. In this case, the locking body may be provided in an elastically deformable form on the moving tab so as to be engaged with the operating portion (500), or may be installed to be elastically supported.

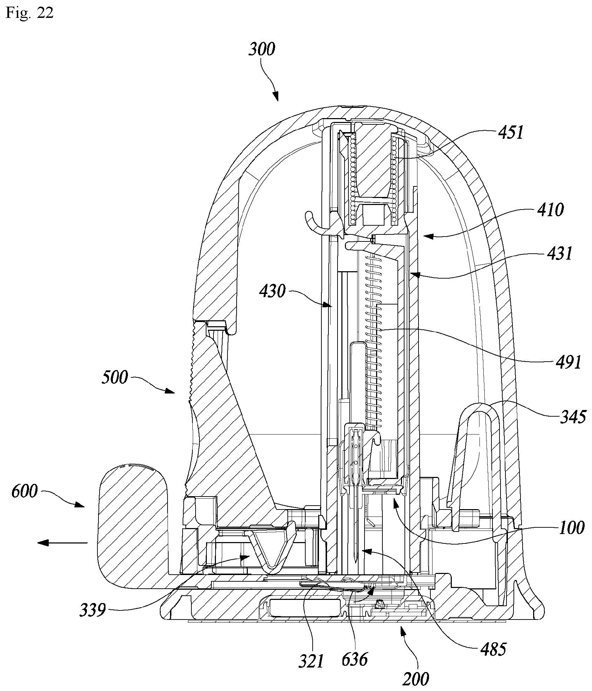

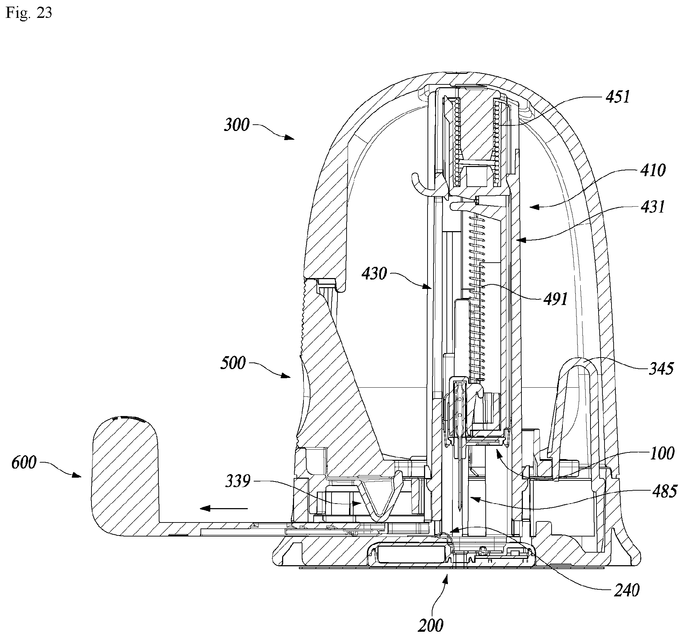

Hereinafter, the process of attaching the medical device (20) to the skin of a user using the applicator (30) according to an embodiment of the present disclosure will be described with reference to FIGS. 22 to 28.

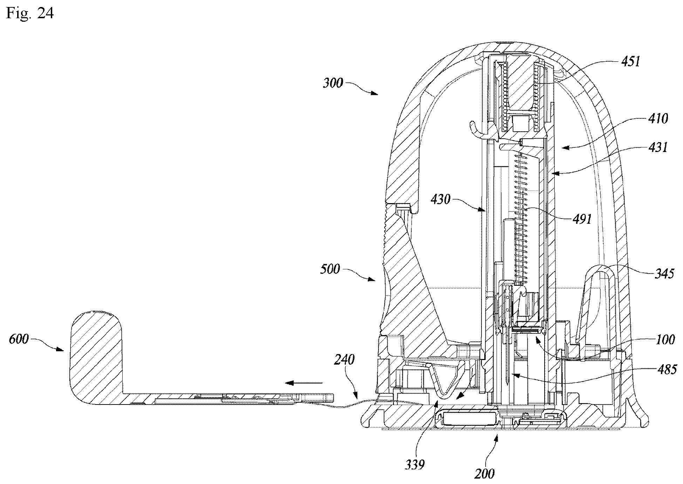

First, as shown in FIGS. 22 to 24, the protective sheet (240) is removed using the moving tab (600). When the moving tab 600 is pulled by a user and moved to the removal position, the moving tab (600) can remove the protective sheet (240) from the adhesive layer (230) by pulling the protective sheet (240) in a direction away from the adhesive layer (230) of the base unit (200). As shown in FIG. 24, when the moving tab (600) moves to the removal position, the locking body (339) is released from the moving tab (600). At this time, the locking body (339) is returned to the initial position so as to be released from engagement with the operating portion (500), and the operating portion (500) is switched to a movable state by a user.

In this way, as the operating portion (500) is switched to an operable state when the moving tab (600) moves to the removal position, the applicator (30) can be operated after the protective sheet (240) is removed from the base unit (200). Accordingly, the sensor unit (100) can move toward the base unit (200) after the protective sheet (240) is removed from the base unit (200).

When the sensor unit (100) moves to the second position in a state in which the insertion unit (430) operates without the protective sheet (240) being completely separated, there may be a problem in which the protective sheet (240) is caught between the sensor unit (100) and the base unit (200), or the sensor unit (100) is coupled with the base unit (200) without the protective sheet (240) being completely separated. The applicator (30) according to an embodiment allows the separation of the protective sheet (240) to be completed before the sensor unit (100) moves from the first position, thereby preventing the sensor unit (100) from reaching the base unit (200) without the protective sheet (240) being separated. Therefore, the risk of malfunction caused by the carelessness of a user can be reduced.

The applicator (30) can be operated in a state in which the bottom portion (312) of the applicator body (300) is in contact with the skin of a user to move the sensor unit (100) toward the skin. When the bottom portion (312) of the applicator body (300) is in contact with the skin of a user, the base unit (200) may be attached to the skin through the adhesive portion (250).

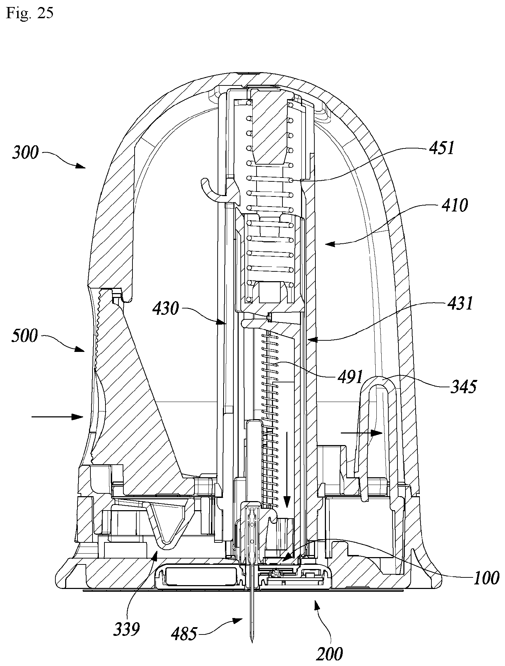

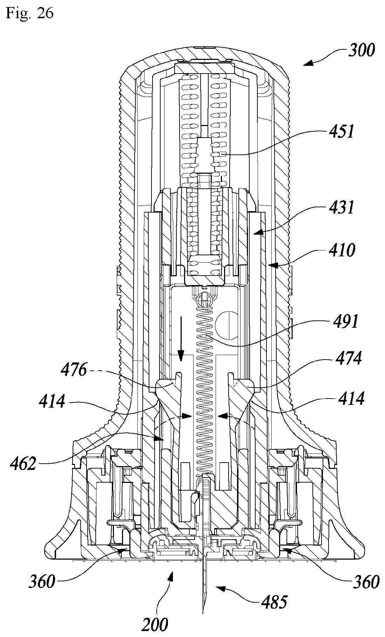

As shown in FIG. 25 and 26, when the operating portion (500) is pressed by a user, the operating portion (500) moves to the activated position while elastically deforming the elastic pressing portion (345). At this time, the backstop member snap (353) of the backstop member (351) is engaged with the backstop detent (528) of the operating portion (500), so that the operating portion (500) is locked in the activated position. As the operating portion (500) moves to the activation position, the shuttle (431) moves away from the stopper portion (520) and moves to the second position by the forward elastic member (451). As the shuttle (431) moves to the second position, the needle (485) and the sensor (110) are inserted into the skin of a user, and the sensor unit (100) is attached to the base unit (200) by the adhesive layer (230). When the shuttle (431) moves to the second position, the trigger (474) of the carrier (462) comes into contact with the release portion (414) of the column member (410). At this time, the carrier wing (471) of the carrier (462) is elastically deformed, thereby disengaging the carrier latch (476) from the shuttle detent (436).

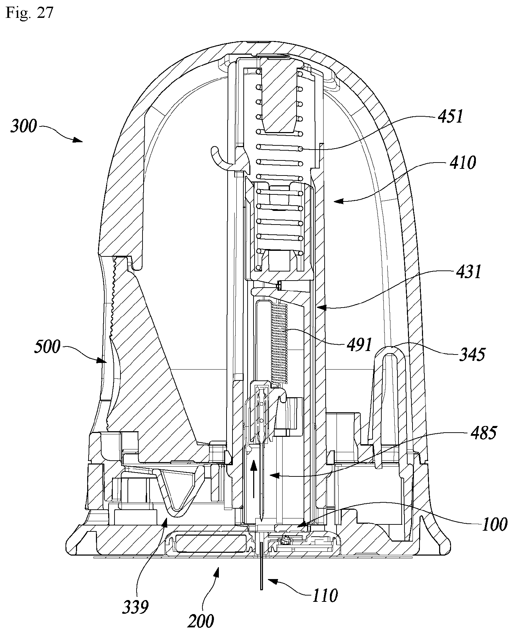

When the carrier (462) is disengaged from the shuttle (431), the carrier (462) is moved in a direction away from the skin of a user by the backward elastic member (491), as shown in FIG. 27. At this time, the needle (485) comes out of the skin and the medical device (20) remains attached to the skin by the adhesive portion (250). While the needle (485) retracts, the shuttle bottom portion (440) of the shuttle (431) located in the second position remains in contact with the sensor unit (100). In addition, the pressing protrusion (442) of the shuttle (431) can remain inserted into the sensor unit groove (138) of the sensor unit (100).

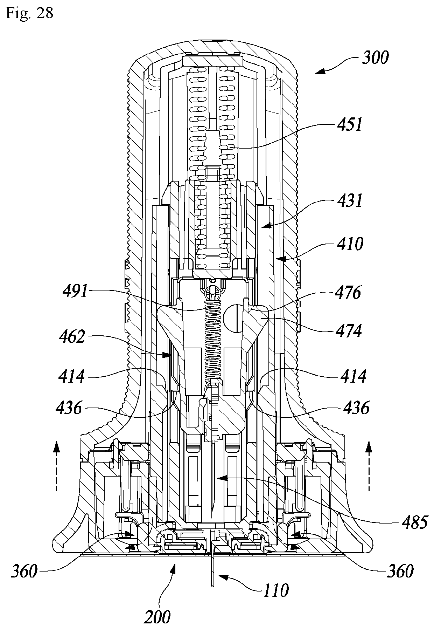

After the sensor (110) is inserted into the skin of a user, the applicator body (300) may be separated from the medical device (20) attached to the skin. In a state in which the operating portion (500) is locked in the activated position, the locking hook (360) engaged with the base unit (200) can be tilted. Therefore, as shown in FIG. 28 , when a user lifts the applicator body (300) from the medical device (20), the locking hook (360) may be tilted and disengaged from the medical device (20).

As described above, in the applicator assembly (10) according to the embodiment of the present disclosure, after a user manipulates the moving tab (600) to remove the protective sheet (240) of the base unit (200), the insertion unit (430) may operate to move the sensor unit (100) toward the base unit (200). Therefore, a malfunction caused by the carelessness of the user may be prevented and safe use may be possible.

In addition, the applicator assembly (10) according to the embodiment of the present disclosure is locked in the activated position without returning to the deactivated position after the operating portion (500) moves to the activated position in which the insertion unit (430) is operated by a user. Therefore, the applicator (30) that has been used once cannot be reused and can be safely discarded.

Although the present disclosure has been described above with preferred examples, the scope of the present disclosure is not limited to the form described and shown above.

For example, the drawing shows that a sensor unit is removably coupled to a carrier which is coupled to a needle and moves from a first position to a second position, but the sensor unit can be detachably coupled to the shuttle or a separate member provided in the shuttle and can move with the shuttle.

Additionally, the drawing shows that the medical device (20) includes a sensor (110) that is inserted into the skin of a user and detects an analyte in the skin, but the medical device may have various other configurations. As another exemplary embodiment, the medical device may take various other configurations having an insertion portion in which at least a portion is inserted into the skin of a user other than the sensor (110).

Additionally, in the drawing, the operating portion (500) shows that a button portion (510) and a stopper portion (520) are integrally formed, but the button portion and the stopper portion may be made of separate parts. In this case, when a user manipulates the button portion, the stopper portion can operate the insertion unit in conjunction with the button portion. The button portion and the stopper portion can be installed on the applicator body so that they can each move in various directions. Additionally, the backstop portion (350) may be configured to engage with at least a portion of the operating portion, that is, one of the button portion and the stopper portion so as to lock one of the button portion and the stopper portion to the activated position. Additionally, when the button portion and the stopper portion are each formed of separate parts, the locking unit (338) may be provided to engage with either the button portion or the stopper portion to restrict the movement of either the button portion or the stopper portion.

Additionally, the drawing shows that the moving tab for removing the protective sheet is configured to move the locking unit (338), but the moving tab may be installed so as not to affect the movement of the locking unit (338). In this case, the applicator may include separate components for moving the locking unit (338). In another exemplary embodiment, the locking unit may be omitted.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

The present invention further may be directed to one or more of the following items:
Item 1: An applicator for a medical device for attaching the medical device to skin of a user, comprising: an applicator body; an insertion unit installed at the applicator body to insert an insertion portion of the medical device, which includes an insertion portion configured to be at least partially insertable into the skin, into the skin of the user; an operation portion installed at the applicator body such that at least a part of the operation portion is movable from a deactivated position to an activated position operating the insertion unit; and a backstop portion configured to lock at least the part of the operation portion in the activated position so as to prevent at least the part of the operation portion from returning from the activated position to the deactivated position.
Item 2: The applicator according to item 1, wherein the backstop portion comprises a backstop detent provided at one of the applicator body and the operation portion, and a backstop member provided at an other one of the applicator body and the operation portion so as to be engaged with the backstop detent after the operation portion moves to the activation position.
Item 3: The applicator according to item 2, wherein the backstop member comprises a backstop member body provided to be elastically deformable at the other one of the applicator body and the operation portion, and a backstop member snap provided at one side of the backstop member body so as to be engaged with the backstop detent.
Item 4: The applicator according to item 3, wherein the one of the applicator body and the operation portion is provided with an accommodation groove in which at least a portion of the backstop member snap is accommodated when the operation portion is located in the deactivated position.
Item 5: The applicator according to item 1, wherein the operation portion is configured to be movable in a direction of intersecting a direction of movement of the insertion portion by the insertion unit.
Item 6: The applicator according to item 1, wherein the insertion unit includes a shuttle to which the insertion portion is detachably coupled, and movably installed at the applicator body to move the insertion portion from a first position where the insertion portion is away from the skin of the user to a second position where the insertion portion is inserted into the skin of the user, and a forward elastic member configured to provide a moving force to the shuttle to move the shuttle to the second position, and wherein the operating portion includes a button portion exposed to an outside of the applicator body; and a stopper portion configured to be movable from the deactivated position to the activated position by the button portion, be engageable with the shuttle in the deactivated position to prevent the shuttle from moving from the first position, and be disengageable from the shuttle in the activated position to allow the shuttle to move to the second position, and wherein the backstop portion is configured to lock the stopper portion in the activated position.
Item 7. The applicator according to item 6, wherein the insertion unit includes a needle configured to be insertable into the skin of the user together with the insertion portion, a carrier coupled to the needle and movably installed to the shuttle, and a backward elastic member configured to apply an elastic force from the second position to the first position with respect to the carrier, and wherein the shuttle includes a fixing groove into which a portion of the backward elastic member is inserted, a contact surface where a portion of the backward elastic member, which is inserted into the fixing groove, contacts, and a barrier portion protruding from the contact surface to prevent the portion of the backward elastic member in contact with the contact surface from falling out from the fixing groove.
Item 8: The applicator according to item 1, comprising: a locking hook tiltably coupled to the applicator body to be engaged with the base unit to detachably couple the base unit, attachable to the skin of the user, to the applicator body for the insertion portion to be coupled, wherein the operation portion is configured to restrain movement of the locking hook so that the locking hook is not tilted when at least the part of the operation portion is located in the deactivated position, and release restraint from the locking hook when at least the part of the operation portion is located in the activated position.
Item 9: The applicator according to item 8, wherein the operation portion includes: a button portion exposed to an outside of the applicator body; a stopper portion configured to move in a direction of intersecting a moving direction of the insertion portion by the button portion; and a stopper protrusion extending from the stopper portion toward the locking hook to contact one side of the locking hook and restrain the movement of the locking hook.
Item 10: The applicator according to item 1, comprising: a locking body installed at the applicator body to be engaged with at least the part of the operation portion or disengaged from at least the part of the operation portion; and a moving tab linearly movably coupled to the applicator body to move from a locking position, where at least a portion of the moving tab is located inside the applicator body, to a removal position, where at least the portion of the moving tab is drawn outward from the locking body, and wherein the moving tab contacts the locking body at the locking position to deflect the locking body to be engaged with at least the part of the operation portion, and wherein the locking body is disengaged from the operation portion when the moving tab moves to the removal position.
Item 11: An applicator assembly comprising: an applicator body; a medical device including an insertion portion configured to be at least partially insertable into skin of a user and detachably coupled to the applicator body; an insertion unit installed at the applicator body to insert the insertion portion into the skin of the user; an operation portion installed at the applicator body so that at least a part of the operation portion is movable from a deactivated position to an activated position operating the insertion unit; and a backstop portion configured to lock at least the of the operation portion in the activated position to prevent at least the part of the operation portion from returning from the activated position to the deactivated position.

## Claims

1. An applicator (30) for inserting at least a portion of a sensor unit (100) configured to measure biometric information of a user into skin of the user, the applicator (30) comprising:
an applicator body (300);
an insertion unit (430) coupled to the applicator body (300) and configured to move the sensor unit (100) along a first direction; and
an operating portion (500) coupled to the applicator body (300) and configured to move from a deactivated position to an activated position to operate the insertion unit (430),
wherein the insertion unit (430) comprises a shuttle (431) configured to move together with the sensor unit (100) along the first direction in response to the operating portion (500) being located in the activated position.

2. The applicator (30) of claim 1,
wherein, when the operating portion (500) is located at the deactivated position, the shuttle (431) is restricted from moving in the first direction by contacting the operating portion (500), and
when the operating portion (500) is located at the activated position, the shuttle (431) is configured to move in the first direction as the contact with the operating portion (500) is released.

3. The applicator (30) of claim 2,
wherein the operating portion (500) comprises
a stopper portion (520) disposed inside the applicator body (300) and configured to contact the shuttle (431).

4. The applicator (30) of claim 3,
wherein, when the operating portion (500) is in the deactivated position, the stopper portion (520) supports the shuttle (431) to restrict movement of the shuttle (431) in the first direction, and
when the operating portion (500) is in the activated position, the stopper portion (520) is spaced apart from the shuttle (431) to allow movement of the shuttle (431) in the first direction.

5. The applicator (30) of any one of the preceding claims,
wherein the operating portion (500) is configured to move in a second direction intersecting the first direction.

6. The applicator (30) of any one of the preceding claims,
wherein the insertion unit (430) comprises
a forward elastic member (451) configured to provide a moving force to the shuttle (431) so that the shuttle (431) moves in the first direction.

7. The applicator (30) of claim 6,
wherein, when the operating portion (500) is in the deactivated position, the forward elastic member (451) is elastically deformed while the shuttle (431) is in contact with the operating portion (500), and
when the operating portion (500) is in the activated position, the forward elastic member (451) applies an elastic force to the shuttle (431) in the first direction as the shuttle (431) is released from contact with the operating portion (500).

8. The applicator (30) of any one of the preceding claims, further comprising
a column member (410) coupled to the applicator body (300) and supporting the insertion unit (430),
wherein the shuttle (431) is movably disposed inside the column member (410).

9. The applicator (30) of claim 8,
wherein the shuttle (431) is configured to move from a first position to a second position along the first direction, and
the column member (410) comprises a blocking portion (420) configured to fix the shuttle (431) at the second position after the shuttle (431) moves to the second position.

10. The applicator (30) of any one of the preceding claims,
wherein the insertion unit (430) comprises:
a needle (485) configured to be inserted into the skin of the user together with at least a portion of the sensor unit (100);
a carrier (462) coupled to the needle (485) and movably disposed to the shuttle (431); and
a backward elastic member (491) configured to apply a moving force to the carrier (462) in a third direction opposite to the first direction,
and wherein the shuttle (431) comprises:
a fixing portion groove (445) into which a portion of the backward elastic member (491) is inserted;
a contact surface (446) contacted by the portion of the backward elastic member (491) inserted into the fixing portion groove (445); and
a barrier portion (447) protruding from the contact surface (446) to prevent the portion of the backward elastic member (491) in contact with the contact surface (446) from falling out from the fixing portion groove (445).
